# EUROPEAN PATENT APPLICATION

(11) **EP 3 607 881 A1**
(43) Date of publication of application: **12.02.2020**
(21) Application number: 19190089.3
(22) Date of filing: 05.08.2019
(51) Int. Cl.: A61B 5/055, A61B 5/026, A61B 18/04, A61B 18/14

(54) **BALLOON POSITIONING USING MAGNETIC RESONANCE IMAGING (MRI) BLOOD FLOW MEASUREMENTS**

(30) Priority: 06.08.2018 US 201816055337
(71) Applicant: Biosense Webster (Israel) Ltd., Yokneam, 2066717 (IL)
(72) Inventor: GLINER, Vadim, 2066717 Yokneam (IL)
(74) Representative: Small, Gary James

(57) **Abstract**

A method for positioning a balloon, which is fitted at a distal end of a catheter, at an ostium of a blood vessel of a patient, includes attempting to achieve circumferential physical contact between the balloon and an entire circumference of the ostium. One or more slices across the ostium are imaged with magnetic resonance imaging (MRI), and the extent of blood flow along the circumference of the ostium is estimated using the MRI. Based on the estimated extent of blood flow, an extent of the circumferential physical contact between the balloon and the ostium is indicated to a user.

## Description

### FIELD OF THE INVENTION

The present invention relates generally to guidance techniques of medical probes, and particularly to guidance of balloon catheters using MRI.

### BACKGROUND OF THE INVENTION

Various techniques for MRI guidance of intra-body probes were previously proposed. For example, U.S. Patent Application Publication 2010/0312096 describes MRI guided cardiac interventional systems that are configured to generate dynamic (interactive) visualizations of patient anatomy and medical devices during an MRI-guided procedure and may also include at least one user selectable 3D volumetric (tissue characterization) map of target anatomy, e.g., a defined portion of the heart. In some embodiments, image slices can be aligned to allow visualization of the device upon tissue contact or activation of ablation energy to allow visualization of the device (e.g., catheter), the device-tissue interface and/or the (myocardium) tissue while receiving the therapy, e.g., ablative energy. Segmented Magnetic Resonance Angiography (MRA) imaging volumes of a patient can be used to generate a vasculature tissue characteristic map which may indicate areas of increased blood flow and/or larger and smaller channels within the vasculature structure.

As another example, U.S. Patent Application Publication 2011/0028829 describes a system and method for non-contrast enhanced pulmonary vein magnetic resonance imaging that substantially suppresses the signal from cardiac tissue adjacent to the left atrium and pulmonary vein is provided. Significant conspicuity of the left atrium and pulmonary vein versus adjacent anatomical structures is produced. In this manner, more accurate measurements of pulmonary vein ostia size are facilitated, as well as more accurate registration of imaging volumes with a radiofrequency ablation catheter during pulmonary vein isolation procedures. In addition, more robust three-dimensional volume views of the left atrium and pulmonary vein are produced without the administration of contrast agents.

### SUMMARY OF THE INVENTION

An embodiment of the present invention provides a method including positioning a balloon, which is fitted at a distal end of a catheter, at an ostium of a blood vessel of a patient, attempting to achieve circumferential physical contact between the balloon and an entire circumference of the ostium. One or more slices across the ostium are imaged with magnetic resonance imaging (MRI), and the extent of blood flow along the circumference of the ostium is estimated using the MRI. Based on the estimated extent of blood flow, an extent of the circumferential physical contact between the balloon and the ostium is indicated to a user.

In some embodiments, the method includes positioning the balloon at an ostium of a pulmonary vein.

In some embodiments, the method includes performing, based on the indicated extent of the circumferential physical contact, a treatment using the balloon.

In an embodiment, the method includes performing radiofrequency (RF) ablation.

In another embodiment, the method includes performing cryogenic ablation.

In an embodiment, the method includes performing laser ablation.

In some embodiments, the method includes applying an MRI protocol that does not require injection of contrast agent to show blood flow.

In some embodiments, the method includes acquiring a sequence of the slices over time.

There is additionally provided, in accordance with an embodiment of the present invention, a medical system, including an interface and a processor. The interface is configured to receive one or more magnetic resonance imaging (MRI) slices acquired by an MRI system across an ostium of a blood vessel of a patient, while a balloon, which is fitted at a distal end of a catheter, is positioned at the ostium and attempts to achieve circumferential physical contact between the balloon and an entire circumference of the ostium. The processor is configured to estimate, using the MRI system, an extent of blood flow along the circumference of the ostium, and, based on the estimated extent of blood flow, indicate to a user an extent of the circumferential physical contact between the balloon and the ostium.

The present invention will be more fully understood from the following detailed description of the embodiments thereof, taken together with the drawings in which:

### BRIEF DESCRIPTION OF THE DRAWINGS

Fig. 1 is a schematic, pictorial illustration of a catheter-based position tracking and ablation system, in accordance with an embodiment of the present invention;
Fig. 2 is a schematic, pictorial illustration of an ablation balloon positioned at an ostium of a pulmonary vein, in accordance with an embodiment of the present invention; and
Fig. 3 is a flow chart that schematically illustrates a method for balloon positioning using magnetic resonance imaging (MRI) flow measurements, in accordance with an embodiment of the present invention.

### DETAILED DESCRIPTION OF EMBODIMENTS

### OVERVIEW

An efficient balloon treatment procedure of a blood-filled organ, such as an ostium of a blood vessel, is optimally performed with the entire circumference of the balloon in physical contact with the ostium tissue, when the balloon treatment can be simultaneously performed over the entire circumference of the ostium. For example, in radiofrequency (RF) ablation treatment, such optimal balloon positioning allows for a "one-shot" ablation, in which all RF electrodes are triggered to ablate surrounding ostium tissue at the same time. For example, a pulmonary vein (PV) isolation procedure, the optimal position of the ablation balloon is where all of the electrodes that are disposed over a circumference of the balloon are in physical contact with the ostium.

If, for example, the balloon treatment is being performed in a procedure where a fluoroscopic image can be taken, correct positioning of the balloon may be confirmed by injecting a contrast agent into the blood. The physician can then see that the balloon effectively "seals off" the ostium (e.g., of a PV).

However, in a magnetic resonance imaging (MRI) procedure, fluoroscopy is generally not available, and while an MRI contrast agent may still be used, it is not well imaged, and, moreover, the MRI system does not image the agent in real time. In this case correct positioning of the balloon may be confirmed by verifying, using impedance measurements, that all of the electrodes have good contact with, for example, the ostium tissue. Impedance measurements, however, are unreliable, especially for patients with problematic vein tissue (e.g., scarred or with fatty surface deposits), so these measurements may not give a valid indication of good contact.

Embodiments of the present invention that are described hereinafter utilize one of several possible protocols for use with an MRI procedure, which are capable of measuring and visualizing blood flow without the injection of a contrast agent. An example of such a protocol is a phase-contrast cine (PC) MRI. PC-MRI provides blood flow information from the entire cross-sectional area of a target ostium. Based on this information, a processor estimates the extent of blood flow through a circumference of the ostium. A detailed description of a PC-MRI protocol is available in numerous publications, such as in a journal paper by Valsangiacomo et al., titled "Phase-contrast MR assessment of pulmonary venous blood flow in children with surgically repaired pulmonary veins," Pediatric Radiology, Volume 33, Issue 9, September, 2003, pages 607-613. Practically, one can use a commercial package for MRI systems, which provides phase-contrast flow and velocity imaging in cardiovascular MRI, to quantify blood flow, such as the *syngo.via* software for MRI provided by Siemens.

Any blood vessel in any anatomical location and orientation can be imaged using MRI in a desired plane (i.e., slice). For accurate measurement of blood flow over an entire circumference of an ostium of a PV, the slice should be perpendicular to the pulmonary vein at its ostium, as described below.

In some embodiments, a user, such as a physician, positions a balloon, which is fitted at a distal end of a shaft for insertion into an organ of a patient, at an ostium of a blood vessel of a patient. The physician operates a magnetic resonance imaging (MRI) system to image one or more slices across the ostium, so as to estimate the blood flow in the slices. Based on the estimated blood flow, a processor indicates to the physician the extent of the circumferential physical contact between the balloon and surrounding tissue of the ostium. A complete blockage of blood flow at a certain point around the ostium is indicative of good physical contact at that point, and *vice versa.*

In some embodiments, the physician navigates the balloon in the left atrium of a heart using a position-tracking system. The tracking system uses, for example, a magnetic location sensor that is included in a distal end of the shaft, just proximally to the balloon. The physician maneuvers the balloon to block an ostium of a PV. Next, the physician uses an MRI system with an MRI protocol that is suited for demonstrating blood flow, in order to image an MRI slice across the ostium. In an embodiment, a processor analyzes the MRI slice to indicate the quality (e.g., extent) of physical contact between the balloon and ostium tissue, based on the demonstrated blood flow. As noted above, lack of blood flow is indicative of good contact, and *vice versa.*

Repeated slices can be acquired approximately every two to three seconds, so that the balloon can be maneuvered relative to the ostium until there is substantially no flow at any point on the circumference of the balloon, wherein no flow corresponds (e.g., as a processor indicates) to good physical contact.

In some embodiments, even if there is not a complete circumferential sealing of the ostium, the MRI slices show which ablation electrodes have no flow. The electrodes are identifiable (e.g., using the location sensor and/or electrical signals through the electrodes), and the ablation radiofrequency generator is configured to provide ablation current only to those electrodes having no flow.

The disclosed technique eliminates the need to use an MRI contrast agent, such as one based on gadolinium, which, as noted above, is hard to apply in an accurate manner due to constraints over timing of the injection of the contrast agent, and which is known to be associated with potential health risks. Thus, the disclosed technique for balloon positioning using MRI flow measurements may enable a physician to perform a more effective and safer balloon ablation procedure under MRI imaging.

### SYSTEM DESCRIPTION

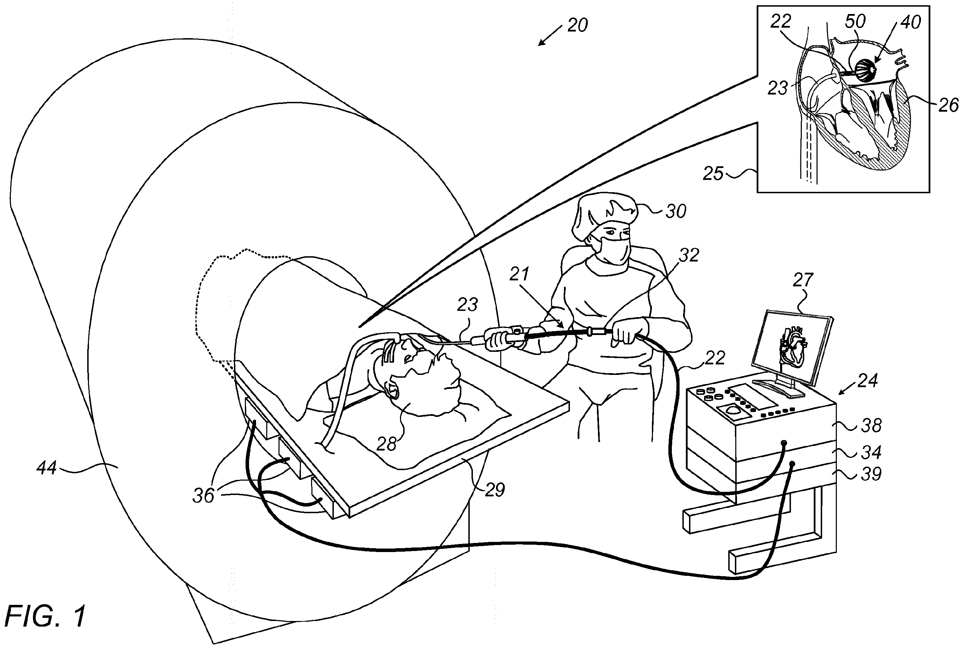

Fig. 1 is a schematic, pictorial illustration of a catheter-based position tracking and ablation system 20, in accordance with an embodiment of the present invention. System 20 is used to determine the position of a balloon 40, seen in an inset 25 fitted at a distal end of a shaft 22. Typically, balloon 40 is used for ablating cardiac tissue, for example, at an ostium of a PV in the left atrium.

Balloon 40 is navigated by a physician 30 into a heart 26 of a patient 28 via the vascular system. In the pictured example, physician 30 inserts shaft 22 of a catheter 21 through a sheath 23, while manipulating shaft 22 using a manipulator 32 near the proximal end of the catheter. As shown in an inset 25, a magnetic position sensor 50 is contained within the distal end of shaft 22.

The proximal end of catheter 21 is connected to a control console 24. Console 24 comprises a processor 39, typically a general-purpose computer, with suitable front end and interface circuits 38 for receiving signals from catheter 21, as well as for applying energy via catheter 21 to ablate tissue in heart 26 and for controlling the other components of system 20. Console 24 also comprises a driver circuit 34, configured to drive magnetic field generators 36. Console 24 drives a display 27, which shows the distal end of the catheter position inside heart 26.

During navigation of distal end 22 in heart 26, console 24 receives position signals from sensor 50 in response to magnetic fields from external field generators 36 of a magnetic catheter-based position tracking system. Magnetic field generators 36 are placed at known positions external to patient 28, e.g., below a table 29 upon which the patient is lying.

The method of position sensing using external magnetic fields is implemented in various medical applications, for example, in the CARTO™ system, produced by Biosense Webster, and is described in detail in U.S. Patents 5,391,199, 6,690,963, 6,484,118, 6,239,724, 6,618,612 and 6,332,089, in PCT Patent Publication WO 96/05768, and in U.S. Patent Application Publications 2002/0065455 A1, 2003/0120150 A1 and 2004/0068178 A1, whose disclosures are all incorporated herein by reference.

As further seen, patient 28 lies inside a bore of an MRI system 44. In some embodiments, processor 39 is configured to receive MRI images of slices of an ostium of a pulmonary vein, which show blood flow from the PV into the left atrium, while balloon 40 is positioned against the ostium. Based on the demonstrated blood flow, processor 41 indicates the quality of the physical contact between the balloon and ostium tissue. In an embodiment, processor 41 indicates the extent to which the balloon is in full contact (i.e., over an entire balloon perimeter) with ostium tissue.

In some embodiments, when system 20 does not include the magnetic position tracking sub-system, processor 39 uses an electrical catheter-based position tracking method, in which electrical position-signals are received from intra-cardiac electrodes, such as electrodes disposed over balloon 40, and/or from the surface electrodes that are attached to the skin of patient 28. Processor 39 uses the electrical position signals to estimate a position of balloon 40, e.g., using an Active Current Location (ACL) method. The ACL method is implemented in various medical applications, for example in the CARTO™ system, produced by Biosense-Webster Inc. (Irvine, California) and is described in detail in U.S. Patents 7,756,576, 7,869,865, and 7,848,787, whose disclosures are all incorporated herein by reference.

Processor 39 typically comprises a general-purpose computer, which is programmed in software to carry out the functions described herein. The software may be downloaded to the computer in electronic form, over a network, for example, or it may, alternatively or additionally, be provided and/or stored on non-transitory tangible media, such as magnetic, optical, or electronic memory.

### BALLOON POSITIONING USING MRI BLOOD FLOW MEASUREMENTS

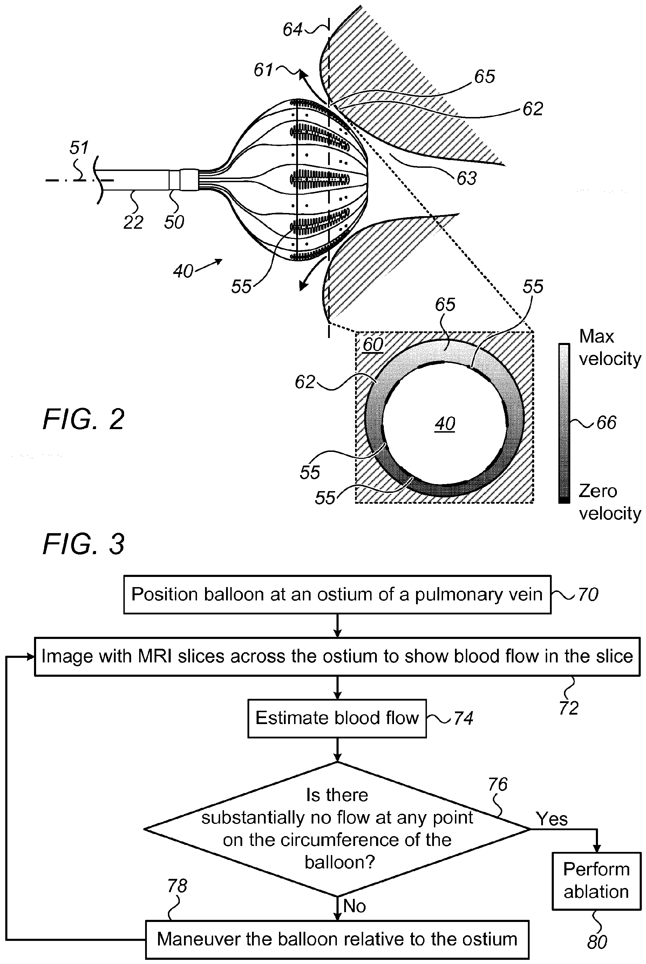

Fig. 2 is a schematic, pictorial illustration of an ablation balloon positioned at an ostium 62 of a pulmonary vein 63, in accordance with an embodiment of the present invention. Balloon 40 is fitted with multiple radiofrequency (RF) ablation electrodes 55, and with magnetic sensor 50. As shown, a distal end of shaft 22 of the catheter is aligned against ostium 62 in such a way that a longitudinal axis 51 of shaft 22 is perpendicular to a plane 64 across ostium 62. Arrows 61 indicate a direction of blood flow over a circumferential gap 65 between balloon 40 and ostium 62 tissue.

An inset 60 illustrates a cross-sectional PC-MRI slice imaged in plane 64 which demonstrates balloon 40, ostium 62, and a largely "circular" gap 65. Subsequently, physician 30 advances the balloon distally, to effectively "seal off" vein 63 (i.e., evenly blocking blood from flowing anywhere through gap 65). In the PC-MRI mode, the amount of blood flow at various points in the slice (e.g., flow rate or velocity) is visualized to the physician, e.g., by a velocity-range legend 66 providing one-directional velocity grayscale (or color scale) encoding through plane 64. This information is also provided to processor 41.

In an embodiment, based on the demonstrated blood flow, processor 41 indicates whether, and at which points, balloon 40 is physically pressed against the circumference of ostium 62. In other words, processor 41 indicates to the physician which of RF electrodes 55 is in contact with ostium 62 tissue. The physician may use this information to maneuver the balloon so that all electrodes are in good physical contact with the vein ostium. In an embodiment, subsequently to "sealing-off" vein 63, physician 30 performs a "one-shot" RF ablation of ostium 62, i.e., simultaneously over an entire circumference of ostium 62.

The example illustration shown in Fig. 2 is chosen purely for the sake of conceptual clarity. Fig. 2 shows only parts relevant to embodiments of the present invention. Other system elements, such as electrophysiological sensing-electrodes disposed over balloon 40 are omitted. In an embodiment, balloon 40 is configured to perform cryogenic ablation of ostium 62. In another embodiment, balloon 40 is configured to perform laser ablation of ostium 62. By nature, initially circumferential gap 65 has a more general curved form than the circular form depicted in inset 60. Yet, when balloon 40 is inflated, and its circular cross-section is pressed against ostium 62, the schematic circular presentation sufficiently indicates that the ostium 62 shape conforms with the balloon shape.

Fig. 3 is a flow chart that schematically illustrates a method for balloon positioning using MRI flow measurements, in accordance with an embodiment of the present invention. The process begins with physician 30 positioning balloon 40 at an ostium 62 of pulmonary vein 63, at a balloon navigation step 70. Next, at an MRI flow imaging step 72, physician 30 acquires a PC-MRI slice across ostium 62 to image blood flow from pulmonary vein 63. Based on the PC-MRI slice, a processor estimates blood flow in the slice, at a blood flow estimation step 74. If, at contact assessment step 76, physician 30 concludes that the quality of physical contact is insufficient, the physician further maneuvers balloon 40 relative to ostium 62, at a maneuvering step 78.

Subsequently, the process loops back to step 72, with the physician acquiring an additional MRI slice across ostium 62 to verify contact quality using the MRI-demonstrated blood flow. If physician 30 concludes that there is substantially no flow at any point on the circumference of the balloon, the physician proceeds to perform a "one-shot" ablation, at a treatment step 80. The process continues until the physician finalizes the ablation procedure.

The example flow-chart shown in Fig. 3 is chosen purely for the sake of conceptual clarity. For example, in alternative embodiments, even if there is no MRI flow indication of complete sealing of the vein, the MRI slices still show which electrodes have no flow. In an alternative treatment step, physician 30 then ablates tissue over part of the circumference, using only electrodes having no flow. The process repeats with additional ostium tissue sections being ablated over an ostium circumference until the entire circumference of ostium 62 is ablated.

Although the embodiments described herein mainly address cardiac applications, the methods and systems described herein can also be used in other applications of ablating blood vessels with an ablation balloon, such as in neurology and in renal denervation.

It will thus be appreciated that the embodiments described above are cited by way of example, and that the present invention is not limited to what has been particularly shown and described hereinabove. Rather, the scope of the present invention includes both combinations and sub-combinations of the various features described hereinabove, as well as variations and modifications thereof which would occur to persons skilled in the art upon reading the foregoing description and which are not disclosed in the prior art. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

### ASPECTS OF THE INVENTION

1. A method, comprising:
   positioning a balloon, which is fitted at a distal end of a catheter, at an ostium of a blood vessel of a patient, attempting to achieve circumferential physical contact between the balloon and an entire circumference of the ostium;
   imaging with magnetic resonance imaging (MRI) one or more slices across the ostium, and estimating using the MRI the extent of blood flow along the circumference of the ostium; and
   based on the estimated extent of blood flow, indicating to a user an extent of the circumferential physical contact between the balloon and the ostium.
2. The method according to aspect 1, wherein positioning the balloon comprises positioning the balloon at an ostium of a pulmonary vein.
3. The method according to aspect 1, and comprising, based on the indicated extent of the circumferential physical contact, performing a treatment using the balloon.
4. The method according to aspect 3, wherein performing the treatment using the balloon comprises performing radiofrequency (RF) ablation.
5. The method according to aspect 3, wherein performing the treatment using the balloon comprises performing cryogenic ablation.
6. The method according to aspect 3, wherein performing the treatment using the balloon comprises performing laser ablation.
7. The method according to aspect 1, wherein imaging the slices comprises applying an MRI protocol that does not require injection of contrast agent to show blood flow.
8. The method according to aspect 1, wherein imaging the slices comprises acquiring a sequence of the slices over time.

## Claims

1. A medical system, comprising:
an interface, which is configured to receive one or more magnetic resonance imaging (MRI) slices acquired by an MRI system across an ostium of a blood vessel of a patient, while a balloon, which is fitted at a distal end of a catheter, is positioned at the ostium and attempts to achieve circumferential physical contact between the balloon and an entire circumference of the ostium; and
a processor, which is configured to:
estimate, using the MRI system, an extent of blood flow along the circumference of the ostium; and
based on the estimated extent of blood flow, indicate to a user an extent of the circumferential physical contact between the balloon and the ostium.

2. The system according to claim 1, wherein the balloon is configured to be positioned at an ostium of a pulmonary vein.

3. The system according to claim 1, wherein the processor is further configured to, based on the indicated extent of the circumferential physical contact, indicate to perform a treatment using the balloon.

4. The system according to claim 3, wherein the balloon is configured to perform radiofrequency (RF) ablation treatment.

5. The system according to claim 3, wherein the balloon is configured to perform cryogenic ablation treatment.

6. The system according to claim 3, wherein the balloon is configured to perform laser ablation treatment.

7. The system according to claim 1, wherein the processor is configured to indicate the extent of physical contact based on an MRI protocol that does not require injection of contrast agent to show blood flow.

8. The system according to claim 1, wherein the MRI system is configured to acquire a sequence of the slices over time.
